# EUROPEAN PATENT SPECIFICATION

(11) **EP 2 193 763 B1**
(45) Date of publication and mention of the grant of the patent: **30.05.2018**
(21) Application number: 08830009.0
(22) Date of filing: 02.09.2008
(51) Int. Cl.: A61F 2/28, A61L 27/00

(54) **ARTIFICIAL BONE**
KÜNSTLICHER KNOCHEN
OS ARTIFICIEL

(30) Priority: 12.09.2007 JP 2007237051
(43) Date of publication of application: 09.06.2010
(73) Proprietor: Kuraray Co., Ltd., Okayama 710-0801 (JP)
(72) Inventor: KIMURA, Naoko, Osaka 530-8611 (JP); KUWAYAMA, Tomoya, Tokyo 100-8115 (JP); NINOMIYA, Mototsugu, Kurashiki-shi Okayama 710-0801 (JP)
(74) Representative: J A Kemp
(86) International application number: PCT/JP2008/065727
(87) International publication number: WO 2009/034876

(56) References cited:
- EP-A1- 1 537 839
- JP-A- 2002 272 833
- JP-A- 2004 275 202
- JP-A- 2007 152 025
- US-A- 5 141 510
- US-A- 5 531 794
- US-A1- 2004 253 185
- US-B1- 6 187 329

## Description

### Technical Field

The present invention relates to an artificial bone used for bone transplantation and the like. More particularly, the present invention relates to an artificial bone having substantially unidirectionally-oriented pores for the improvement of osteoconductivity.

### Background Art

In general, an artificial bone having a pore structure is widely used to secure osteoconductivity in bone transplantation and the like. As such an artificial bone having a pore structure, for example, a calcium phosphate sintered body wherein many pores are densely distributed three-dimensionally, and a skeleton wall compartmentalizing adjacent pores has linked sphere-like opened pores communicating with them (see patent document 1) is conventionally known. However, the osteoconductivity thereof is not entirely satisfactory. Moreover, it is disclosed that a sintered body having unidirectionally-oriented pores with a diameter of 10 - 500 µm is a ceramic material suitable as an implant material (patent document 2).
patent document 1: JP-B-3470759
patent document 2: JP-B-3858069

### Disclosure of the Invention

### Problems to be Solved by the Invention

Generally, when a material having substantially unidirectionally-oriented pores is used as an artificial bone, the maximum osteoconductivity can be exhibited by implanting the bone while setting the pore orientation in an appropriate direction. However, it is extremely difficult to visually recognize the direction of orientation of fine pores having a diameter of about 10 - 500 µm, and misidentification of the implantation direction of an artificial bone is feared to cause decreased treatment effects such as elongated time necessary for healing and the like. Moreover, since the initial strength of the artificial bone varies depending on the oriented direction of the pores, it is important to implant an artificial bone in an affected part by setting the oriented direction of the pores to a desired direction based on the understanding of the oriented direction of the pores in the artificial bone.

The present invention has been made in view of such situation, and aims to provide an artificial bone capable of easily and accurately confirming the direction of substantially unidirectionally-oriented pores to achieve good osteoconductivity.

US 2004/0253185 discloses a medical device having a medicated ink mark.

US 6,187,329 discloses an implant with a porosity between 1 and 99% which may contain other materials such as colorants.

US 5,141,510 discloses devices with hollowed minute tubes.

US 5,531,794 discloses a device with a tubular passage.

EP 1,537,839 discloses a triphasic prosthetic device. Means of Solving the Problems

The present inventors have conducted intensive studies in an attempt to solve the aforementioned problems and completed the present invention having the following characteristics.

Accordingly, the present invention relates to
(1) an artificial bone comprising substantially unidirectionally-oriented pores, which has a marker showing the orientation direction of said pores, wherein
   the marker comprises a line symbol, a protrusion or a pit marked on the surface of the artificial bone, and
   the diameter of the section vertical to the long axis of said pores is 10 - 500 µm.

### Effect of the Invention

Since the artificial bone of the present invention has a marker showing the direction of orientation of the substantially unidirectionally-oriented pores, for example, a physician can easily and accurately know the direction along which the artificial bone is to be implanted during bone transplantation.

### Brief Description of the Drawings

Fig. 1 is a perspective view schematically showing a first embodiment of the artificial bone of the present invention.
Fig. 2 is a perspective view schematically showing a second embodiment of the artificial bone of the present invention.
Fig. 3 is a perspective view schematically showing a third embodiment of the artificial bone of the present invention.
Fig. 4(a) and Fig. 4(b) are perspective views schematically showing a fourth embodiment (2 embodiments) of the artificial bone of the present invention, Fig. 4(c) and Fig. 4(d) are perspective views schematically showing a fifth embodiment (2 embodiments) of the artificial bone of the present invention, and Fig. 4(e) is a perspective view schematically showing a sixth embodiment of the artificial bone of the present invention.
Figs. 5(a) - (c) are perspective views schematically showing a seventh embodiment (3 embodiments) of the artificial bone of the present invention.

### [Explanation of symbols]

1 artificial bone material (artificial bone main body)
2 pores
3a line symbol
3b protrusion
3c pit
10, 20, 30, 40A - 40E, 50A - 50C, 60A - 60C, 70 artificial bone
41a - 41c dot symbol
42 line symbol
43 arrow symbol
44 triangle symbol
X arrow showing the direction of orientation of substantially unidirectionally-oriented pores

### Best Mode for Carrying out the Invention

The present invention is explained in detail in the following by referring to preferable embodiments thereof.

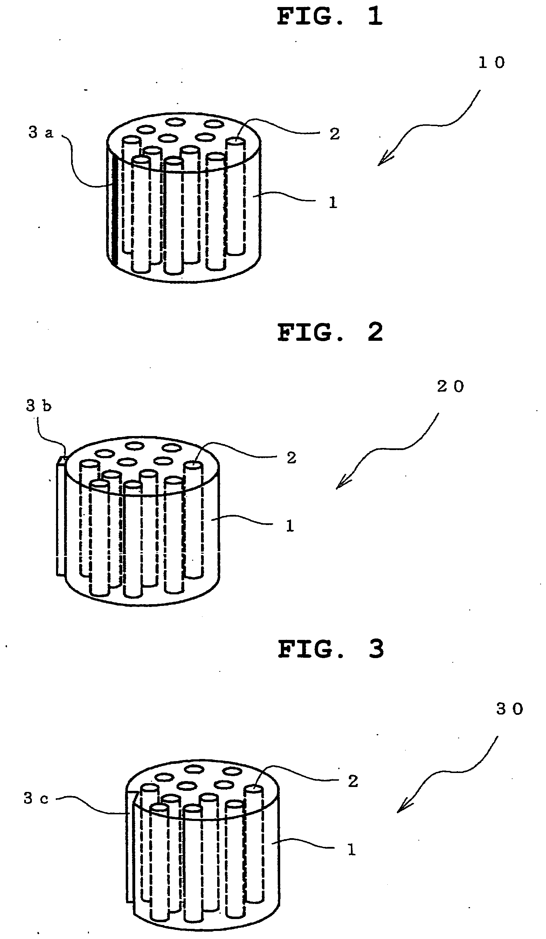

Fig. 1 is a perspective view schematically showing one embodiment of the artificial bone of the present invention.

The artificial bone of the present invention has, as shown by artificial bone 10 of said embodiment, substantially unidirectionally-oriented pores 2 in the inside of the material (artificial bone main body) 1. The pores being "unidirectionally oriented" here mean that pores extending in the uniaxial direction are present and the long axis direction of such pores is arranged to be unidirectional. More specifically, for example, the long axis direction of not less than half, preferably not less than 80%, more preferably not less than 90%, of the pores extending in the uniaxial direction in the material (artificial bone) is aligned unidirectionally. In addition, "substantially unidirectional" means that the long axis of pores are aligned to fall within the crossing angle of 30°.

The material of the artificial bone of the present invention is not subject to any particular limitation. Examples thereof include calcium phosphate, silica, titania, zirconia and the like. Among these, calcium phosphate is preferable. Preferable examples of calcium phosphate include hydroxyapatite, fluorapatite, chlorapatite, tricalcium phosphate, calcium metaphosphate, tetracalcium phosphate, calcium hydrogen phosphate, calcium hydrogen phosphate dihydrate and the like. In the material of the present invention, a part of Ca component of calcium phosphate may be substituted by one or more kinds selected from Sr, Ba, Mg, Fe, Al, Y, La, Na, K, Ag, Pd, Zn, Pb, Cd, H and other rare earths. In addition, a part of (PO₄) component may be substituted by one or more kinds selected from VO₄, BO₃, SO₄, CO₃, SiO₄ and the like. Furthermore, a part of (OH) component may be substituted by one or more kinds selected from F, Cl, O, CO₃, I and Br.

For bone formation, calcium phosphate is more preferably hydroxyapatite, fluorapatite, chlorapatite or tricalcium phosphate, most preferably hydroxyapatite or tricalcium phosphate, and calcium phosphate may form a composite with collagen and the like. Calcium phosphate may be derived from natural mineral, or may be synthesized by various processes, dry processes and the like.

An artificial bone having substantially unidirectionally-oriented pores can be produced by a known method.

When artificial bone 10 in the above-mentioned embodiment is to be produced, for example, a cylindrical container having a brass plate as a basal plate and a vinyl chloride resin as a side wall is prepared. On the other hand, a slurry obtained by dispersing or dissolving hydroxyapatite and, where necessary, an organic binder (e.g., one or more kinds selected from collagen, gelatin, polylactic acid, polyacrylic acid, polyethylene glycol, polyvinyl alcohol and the like) in water is prepared. The slurry is filled in the above-mentioned cylindrical container, and the basal plate is cooled with liquid nitrogen and the like to not higher than the coagulation point of the slurry to form needle-like ice in the slurry from the base. As a different method, a cylindrical container containing a slurry is inserted (immersed) in a refrigerant cooled to not higher than the coagulation point of the slurry by, for example, a suitable power source such as constant-speed motor and the like to form needle-like ice in the slurry from the base of the cylindrical container. In addition, the ice is sublimated and removed under vacuum and, where necessary, the residue is sintered at 1000°C or above to give an artificial bone (having a cylindrical external shape) 10 having substantially unidirectionally-oriented pores 2. When a prism-shaped container, cylindroid-shaped container and the like are used as the container to fill in the above-mentioned slurry, an artificial bone having a prism-like or cylindroid-like external shape and the like can be obtained.

The shape (external shape) of the artificial bone of the present invention is not particularly limited, and it may be a shape other than the above-mentioned cylindrical shape, prism-like shape and cylindroid-like shape. That is, the artificial bone of the present invention encompasses one obtained by processing (post-processing) an artificial bone having a column-like shape into a desired shape, one obtained by joining a plurality of column-like artificial bones and post-processed artificial bones by a suitable joining method and the like. That is, a plurality of bone parts having substantially unidirectionally-oriented pores may be combined.

While the size of the substantially unidirectionally-oriented pores is not particularly limited, the diameter (diameter of section vertical to the long axis of pores) is about 10 - 500 µm, the length (length of pores in the long axis direction) is preferably not less than 5 mm, more preferably not less than 7 mm, further preferably not less than 10 mm. The upper limit of the length is not particularly limited.

The porosity is generally 40 - 90%, and preferably not less than 50%, more preferably not less than 80%, of all pores are constituted with "substantially unidirectionally-oriented pores".

The artificial bone of the present invention has substantially unidirectionally-oriented pores, and a marker showing the pore orientation direction. The marker comprises a line symbol, a protrusion or a pit marked on the surface of the artificial bone.

The artificial bone 10 in the above-mentioned embodiment (Fig. 1) has a line symbol 3a on its surface as a marker showing the orientation direction of unidirectionally-oriented pores 2. Here, the "line symbol" is not particularly limited as to the formation method as long as it can be recognized visually as a "line" on the surface of the artificial bone. One drawn with a dye which is harmless to the body and does not inhibit bone formation is preferable. Examples of the dye which is harmless to the body and does not inhibit bone formation include degradable dyes such as natural dye, food dyes and the like, non-degradable dyes that do not change or denature when implanted in the body and the like. Particularly, a dye having a color clearly different from the color of an artificial bone material (artificial bone main body) is selected. Specific examples include flavonoids such as anthocyanin and the like, carotenoid dyes such as β-carotene and the like, transition metal oxides such as titanium dioxide, iron sesquioxide, copper oxide, cobalt oxide and the like, metals such as gold, platinum, copper and the like, alloys thereof and the like. While the marking method of line symbol 3a is not particularly limited, for example, an inkjet method, a thermosensitive method, a vacuum deposition method, a sputtering method and the like can be employed for marking.

In the present invention, line symbol 3a is not particularly limited as long as the orientation direction of pores 2 can be easily and accurately recognized. As shown in the example of the above-mentioned Fig. 1, it may be a straight line running substantially in parallel with the orientation direction (long axis of pores) of unidirectionally-oriented pores 2, an arrow (with an arrow direction toward the orientation direction of unidirectionally-oriented pores 2) and the like. The line symbol 3a preferably has a line width appropriate for visual recognition, and the line width is preferably about 0.01 - 0.5 mm.

Figs. 2 and 3 show other embodiments of the artificial bone of the present invention. The artificial bone 20 shown in Fig. 2 has, as a marker showing the orientation direction of substantially unidirectionally-oriented pores 2, protrusion 3b running substantially in parallel with the orientation direction of pores 2 on the surface thereof. The protrusion 3b may be formed with a material different from the artificial bone material (artificial bone main body) 1. However, it is preferably the same material as the artificial bone material (artificial bone main body) 1. When the material is the same, a protrusion can be formed by cutting processing of the artificial bone by a cutting machine and the like. The artificial bone 30 shown in Fig. 3 has, as a marker showing the orientation direction of substantially unidirectionally-oriented pores 2, pit 3c running substantially in parallel with the orientation direction of pores 2 on the surface thereof. While the marking method of pit 3c is not particularly limited, for example, a method including cutting processing of the surface of the artificial bone by a cutting machine, or laser radiation and the like by a laser processing machine and the like, and the like can be mentioned. Such protrusion 3b and pit 3c are generally substantially linear protrusion (pit) with the axial center substantially in parallel with the pore orientation direction (axial center of pores).

The protrusion 3b and pit 3c as such markers preferably have a protrusion height or pit depth of about 0.1 - 2 mm so that they can be at least recognized visually or by tactile sense. With a height or depth in such range, the sense of concave convex can be obtained with human fingers and the like, and can also be visually recognized since the reflectance of the light at the marked portion on the surface of the artificial bone is different from that of the surrounding area.

In addition, the shape of protrusion 3b and pit 3c is not particularly limited as long as it enables easy and accurate recognition of the pore direction. In the embodiment of Fig. 2, the shape of protrusion 3b is a cuboid (quadratic prism). It may be, for example, a semi-column, a triangular prism and the like. In the embodiment of Fig. 3, pit 3c is a groove having a V-shaped cross section. It may be, for example, a groove having a square U-shaped or semicircle-shaped cross section.

In the artificial bone of the present invention, the marker showing the direction of orientation of substantially unidirectionally-oriented pores may be constituted with a composite of a line symbol and a protrusion, or a line symbol and a pit. That is, for example, it may be protrusion 3b and pit 3c shown in Figs. 2, 3 colored with a dye. Using such composite constitution, a marker enabling recognition by a tactile sense and showing good visual recognizability can be realized.

Moreover, the artificial bone of the present invention also includes, as mentioned above, an artificial bone comprising a plural combinations of bone parts having substantially unidirectionally-oriented pores. In such artificial bone, a marker (line symbol, protrusion or a pit) is attached to each bone part having substantially unidirectionally-oriented pores, whereby the direction of orientation of unidirectionally-oriented pores in each bone part can be known easily.

In the present invention, the size of the marker comprising a line symbol, protrusion or a pit is not particularly limited. For easy recognition, however, a size containing a part with a length of one direction of about 3 - 50 mm is preferable. That is, when a marker is, for example, a linear line symbol shown in Fig. 1, the length of the straight line is preferably about 3 - 50 mm.

A marker in the form of a line symbol or protrusion can be eliminated as necessary by a diamond bar or disk and the like when using the artificial bone (when artificial bone is actually implanted in the body).

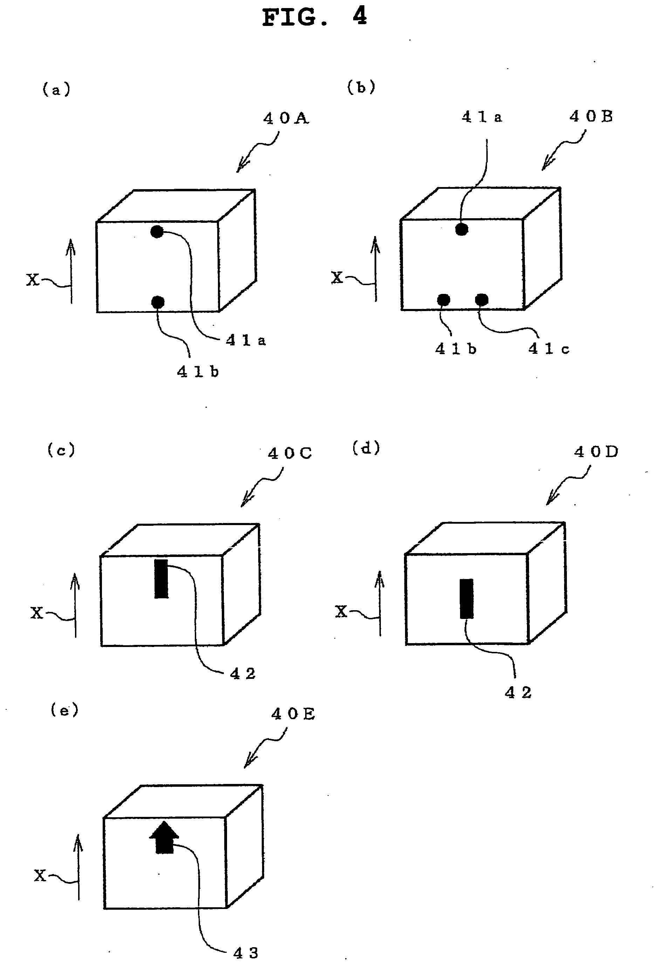

In the present invention, examples of the marker showing the direction of orientation of unidirectionally-oriented pores (hereinafter sometimes to be simply referred to as "pore orientation direction") besides the aforementioned embodiments include those shown in Fig. 4 - Fig. 5. In these Figures, pores are not schematically shown, and the direction of pore orientation is shown with an arrow (arrow X) for convenience.

Figs. 4(a) and (b) show embodiments wherein the surface of an artificial bone is marked with dot symbols. In the artificial bone 40A shown in Fig. 4(a), two dots 41a, 41b are marked on its surface such that the straight line connecting them is substantially in parallel with the pore orientation direction (arrow X). In the artificial bone 40B shown in Fig. 4(b), three dots 41a - 41c are marked on its surface such that a triangle formed by connecting them with a straight line is substantially isosceles triangle or substantially equilateral triangle and the bisector of one given vertex angle (angle of vertex 41a) is substantially in parallel with the pore orientation direction (arrow X). The shape of the dot symbols 41a - 41c is not particularly limited, and may be a circular shape illustrated therein or a shape other than the circular shape.

In the embodiments of artificial bones 40C and 40D shown in Figs. 4(c) and (d), a line symbol 42 is marked on their surfaces. In the artificial bone 10 shown in the aforementioned Fig. 1, line symbol 3a is drawn in the pore orientation direction over the full length from one end to the other end of the surface marked with line symbol 3a. In the artificial bones 40C and 40D, line symbol 42 is drawn short rather than drawing over the full length from one end to the other end of the marked surface. To be specific, artificial bone 40C (Fig. 4(c)) is an embodiment wherein line symbol 42 is drawn in the pore orientation direction on one end side of the marked face, and artificial bone 40D (Fig. 4(d)) is an embodiment wherein line symbol 42 is drawn in the pore orientation direction on the approximately center part of the marked face. As shown in these embodiments, the line symbol does not need to be drawn over the full-length from one end to the other end of the marked face in the pore orientation direction in the present invention, as long as it becomes an index enabling easy recognition of the pore orientation direction, and may be partially drawn in the pore orientation direction of the marked face.

In addition, artificial bone 40E shown in Fig. 4(e) is an embodiment wherein an arrow symbol is marked on its surface, and the arrow direction of arrow symbol 43 is the same as the pore orientation direction (arrow X). In the explanation of line symbol 3a in the aforementioned Fig. 1 (artificial bone 10), an embodiment of a combination of a line symbol and an arrow (i.e., line symbol drawn over the full-length from one end to the other end of the marked face in the pore orientation direction, with one end thereof having an arrow shape) is explained. In the arrow symbol 43 shown in Fig. 4(e), the length in the symbol arrow direction is short and comparatively broad in width as shown therein, which prevents recognition of the symbol as a line symbol.

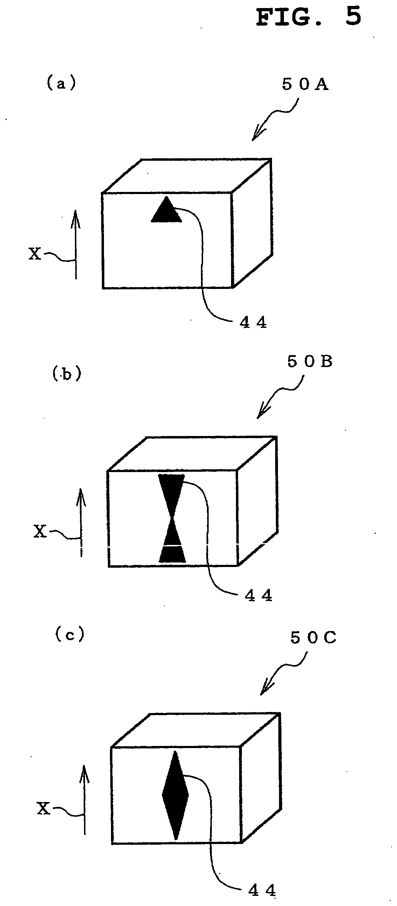

The artificial bones 50A - 50C shown in Figs. 5(a) - (c) are embodiments having triangle symbols 44 as markers on the surfaces thereof, wherein the direction of one vertex angle of triangle of the triangle symbols 44 is the same as the pore orientation direction (arrow X). The shape, number and layout of triangle of the triangle symbol can be changed variously as shown in Figs. 5(a) - (c), and are not particularly limited. The marker may be constituted with 3 or more triangle symbols.

In the artificial bones shown in Figs. 4(a) - (e) and Figs. 5(a) - (c) above, the method of forming dot symbols 41a - 41c, line symbols 42, arrow symbols 43 and triangle symbol 44 and the like as markers showing the pore orientation direction is not particularly limited. A symbol drawn with a dye which is harmless to the body and does not inhibit bone formation is preferable, as in line symbol 3a of artificial bone 10 in the aforementioned Fig. 1. Specific examples of such dye include those mentioned above. In addition, a method of marking the symbol may be any such as an inkjet method, a thermosensitive method, a vacuum deposition method, a sputtering method and the like.

While the size of the dot symbols 41a - 41c, arrow symbol 43, triangle symbol 44 and the like is not particularly limited, a size easily recognized visually, which does not require attachment of an excess amount of dye, is preferable. For example, when the dot symbol has a circular shape, it generally has a diameter of about 0.5 - 3.0 mm, and when the dot symbol has a shape other than a circular shape, it appropriately has a size (area) corresponding to the area of a circle having a diameter of 0.5 - 3.0 mm. In addition, arrow symbol 43, triangle symbol 44 and the like also preferably have a size equivalent to or slightly larger than that of such circular dot symbol.

Such dot symbols 41a - 41c, line symbol 42, arrow symbol 43, triangle symbol 44 and the like may be formed not only by coloring but also by complexing with a pit or protrusion, like the line symbol 3a of artificial bone 10 in the aforementioned Fig. 1.

The specific examples of the artificial bone of the present invention as mentioned above are embodiments wherein the marker showing the pore orientation direction is constituted with a symbol marked on the surface of the artificial bone.

### Examples

The present invention is explained more specifically in the following by referring to Examples.

### [Production Example A of artificial bone]

Ten cylindrical containers (inner diameter 16 mm, height 20 mm without basal plane) made of vinyl chloride resin were directly placed on a discal cooling plate (diameter 120 mm), and a slurry obtained by dispersing or dissolving hydroxyapatite (17 wt%) and gelatin (4 wt%) as an additive in distilled water was filled in the containers by 1 g for each container. By cooling the cooling plate with a freezing apparatus, slurry was cooled at a freezing rate of 0.015 ml/min to allow formation of needle-like ice in the slurry. The thus-obtained frozen body was sublimated and dried under vacuum, and the green body was sintered at 1200°C to give an artificial bone having unidirectionally oriented pores.

### [Production Example B of artificial bone]

A slurry (10 g) obtained by dispersing or dissolving hydroxyapatite (21.8 wt%) and gelatin (4.8 wt%) as an additive in distilled water was filled in a 15 ml centrifugation tube with an inner diameter of about 16 mm (made of polypropylene resin, manufactured by Greiner, Germany) as a container. The container was immersed in an ethyl alcohol bath cooled to -20°C at a rate of 20 mm/h to allow formation of needle-like ice in the slurry. The thus-obtained frozen body was sublimated and dried under vacuum, and the green body was sintered at 1200°C to give an artificial bone having unidirectionally-oriented pores.

### [Embodiment with line symbol]

The artificial bones obtained in Production Examples A and B were cut in a cylindrical shape (ϕ 6 mm, height 8 mm) by a cutting machine, MODELA Pro MDX-650, manufactured by Roland. Thereafter, β-carotene (0.1%) dissolved in ethyl alcohol was used as a dye and a line symbol (line width 0.3 mm, length 8 mm) as shown in Fig. 1 was marked, whereby artificial bones marked with the line symbol were obtained.

### [Embodiment with protrusion]

The above-mentioned artificial bones obtained in Production Examples A and B were cut in a cylindrical shape (ϕ 6 mm, height 8 mm) by a cutting machine, MODELA Pro MDX-650, manufactured by Roland, and a protrusion (substantially cuboid, length 1 mm×width 1 mm, height 8 mm) was simultaneously formed on the cylindrical side face from the basal plane to the top face as shown in Fig. 2, whereby artificial bones with the protrusion were obtained.

### [Embodiment with pit]

The above-mentioned artificial bones obtained in Production Examples A and B were cut in a cylindrical shape (ϕ 6 mm, height 8 mm) by a cutting machine, MODELA Pro MDX-650, manufactured by Roland. Furthermore, a pit (V-shaped groove of cross section, opening width 1 mm, depth 1 mm, height 8 mm) was formed on the cylindrical side surface from the basal plane to the top face as shown in Fig. 3, whereby artificial bones with the pit were obtained.

### [Embodiment with protrusion and line symbol]

The above-mentioned artificial bones obtained in Production Examples A and B were cut in a cylindrical shape (ϕ 6 mm, height 8 mm) by a cutting machine, MODELA Pro MDX-650, manufactured by Roland, and a protrusion (substantially cuboid, length 1mm×width 1mm, height 8 mm) was simultaneously formed on the cylindrical side surface from the basal plane to the top face as shown in Fig. 2. Furthermore, β-carotene (0.1%) dissolved in ethyl alcohol was used as a dye and a line symbol (line width 0.3 mm, length 8 mm) as shown in Fig. 1 was marked on the surface of the protrusion of the artificial bones, whereby artificial bones with the protrusion and line symbol were obtained.

### Industrial Applicability

Since the artificial bone of the present invention contains substantially unidirectionally-oriented pores as well as a marker showing the pore orientation direction, the direction along which the artificial bone is to be implanted can be easily and accurately known during bone transplantation. Hence, physicians can certainly implant an artificial bone having substantially unidirectionally-oriented pores in an appropriate direction during bone transplantation, and therefore, treatment effects are expected to be improved.

## Claims

1. An artificial bone comprising substantially unidirectionally-oriented pores, which has a marker showing the orientation direction of said pores, wherein
the marker comprises a line symbol, a protrusion or a pit marked on the surface of the artificial bone, and
the diameter of the section vertical to the long axis of said pores is 10 - 500 µm.

## Patentansprüche

1. Künstlicher Knochen, der im Wesentlichen unidirektional orientierte Poren umfasst und einen Marker aufweist, der die Orientierungsrichtung der Poren zeigt, wobei
der Marker ein Liniensymbol, einen Vorsprung oder ein Grübchen, die auf der Oberfläche des künstlichen Knochens markiert sind, umfasst und
der Durchmesser des Schnittes vertikal zur Längsachse der Poren 10 bis 500 µm beträgt.

## Revendications

1. Os artificiel comprenant des pores sensiblement orientés de manière unidirectionnelle, qui a un marqueur représentant la direction d'orientation desdits pores, dans lequel :
le marqueur comprend un symbole de ligne, une saillie ou un puits marqué sur la surface de l'os artificiel, et
le diamètre de la section verticale par rapport à l'axe long desdits pores est de 10 à 500 µm.
